# EUROPEAN PATENT SPECIFICATION

(11) **EP 1 526 894 B1**
(45) Date of publication and mention of the grant of the patent: **14.12.2011**
(21) Application number: 03792272.1
(22) Date of filing: 06.08.2003
(51) Int. Cl.: A61P 3/04, A61K 31/216, A61K 31/4439

(54) **USE OF A PPAR-ALPHA AGONIST TO TREAT WEIGHT GAIN ASSOCIATED WITH A PPAR-GAMMA AGONIST TREATMENT**
VERWENDUNG EINES PPAR-ALPHA AGONISTS ZUR BEHANDLUNG VON MIT PPAR-GAMMA AGONIST-BEHANDLUNG ASSOZIERTEN KÖRPERGEWICHTSZUNAHME
UTILISATION D'UN AGONISTE DE PPAR ALPHA POUR TRAITER UNE PRISE DE POIDS LIEE A UN TRAITEMENT PAR LES AGONISTES DE PPAR GAMMA

(30) Priority: 08.08.2002 EP 02291994; 14.11.2002 EP 02292830
(43) Date of publication of application: 04.05.2005
(73) Proprietor: Fournier Laboratories Ireland Limited, Carrightwohill Cork (IE)
(72) Inventor: JUNIEN, Jean-Louis, F-92310 Sevres (FR); EDGAR, Alan, F-21490 Saint-Julien (FR); CHAPUT, Evelyne, F-68510 Sierentz (FR)
(74) Representative: Gillet, Raphaëlle
(86) International application number: PCT/EP2003/008756
(87) International publication number: WO 2004/018041

(56) References cited:
- WO-A-98/05331
- WO-A-02/080936
- CHAPUT ET AL: "Fenofibrate and rosiglitazone lower serum triglycerides with opposing effects on body weight" BIOCHEMICAL AND BIOPHYSICAL RESEARCH COMMUNICATIONS, vol. 271, no. 2, 2000, pages 445-450, XP001151928 cited in the application

## Description

The present invention relates to the use of fenofibrate to treat patients suffering from weight gain associated with a rosiglitazone treatment.

Great advances have been made in the management of diabetes during the past decade. Whereas only one class of oral medications (the sulfonylureas) was available for the treatment of type 2 diabetes in the early 1990s, new classes of oral antidiabetic agents were developed. The thiazolidinedione class of medications was first introduced to the United States when troglitazone was marketed during early 1997. Rosiglitazone, approved by the FDA during the spring of 1999, was the second thiazolidinedione to be marketed in the United States. Similar to troglitazone, rosiglitazone improves insulin sensitivity in patients with Non-Insulin-Dependent Diabetes Mellitus by activating peroxisome proliferator-activated receptor-gamma (PPARγ) receptors in adipose tissues and skeletal muscles.

Non-Insulin-Dependent Diabetes Mellitus (NIDDM) is a form of diabetes where utilization of insulin is not the first line therapy. It occurs predominantly in adults, in whom production of insulin is available for use, yet a defect exists in insulin-mediated utilization and metabolism of glucose and peripheral tissues. For some people with diabetes, a mutation in the genes coding for insulin, for insulin receptor and/or for insulin-mediated signal transduction factors leads to ineffective insulin and/or insulin-mediated effects, impairing the utilization or metabolism of glucose.

The pathophysiology of non-insulin-dependent diabetes mellitus consists of three major components, (1) peripheral insulin resistance; (2) increased hepatic glucose production; and (3) impaired insulin secretion. Intense research has been devoted to each of these areas, independently, in order to determine which abnormality is primary and which are secondary.

When focussing on peripheral insulin resistance, the drug of choice is a thiazolidinedione, which is a type of insulin-sensitizing agent.

The thiazolidinedione chemical series has been shown to reverse insulin resistance in patients with NIDDM and impaired glucose tolerance, and can enhance insulin action in numerous genetic and acquired rodent models of insulin resistance. The antihyperglycemic effects of thiazolidinediones result from the ability to increase insulin dependent glucose disposal and reduce hepatic glucose production. It is believed that, by enhancing insulin action, thiazolidinedione treatment results in euglycemia at a lower circulating insulin level. In this regard, studies in normal and diabetic rodents and human clinical trials have not revealed hypoglycemia as a complication of thiazolidinedione therapy. On the other hand, administration of these drugs to normal or insulin-deficient diabetic animals failed to alter plasma glucose or insulin or glucose tolerance, although insulin sensitivity was nevertheless increased.

The effects of thiazolidinediones on glucose disposal are thought to result from insulin sensitization, indicating an absolute requirement for insulin. Thiazolidinedione treatments are based on the assumption that if peripheral insulin resistance is improved, increased hepatic glucose production and impaired insulin secretion will be alleviated in due course.

It has been observed that rosiglitazone markedly increased body weight gain (E Chaput et al, Biochem Biophys Res Commun 2000 May 10;271(2):445-50). This side effect renders rosiglitazone monotherapy an undesirable prophylactic measure in the treatment of NIDDM.

As already explained, rosiglitazone is a PPARγ activator or agonist. In the present invention, the term agonist or activator is used equally to designate a compound that can activate a PPAR receptor.

PPARγ is a subtype of the PPAR (Peroxisome Proliferator Activated Receptor) family. PPARγ is predominantly expressed in white adipose tissue in rodents. Its expression is induced early during the course of differentiation of several preadipocyte cell lines. In fibroblasts, forced expression of PPARγ in the presence of an agonist such as a thiazolidinedione results in differentiation to an adipocyte phenotype.

Other activators of PPARs are effective drugs to improve the metabolic abnormalities linking hypertriglyceridemia to diabetes, hyperglycemia, insulin-resistance, and atherosclerosis.

Among them, fibrates can be cited as PPARα activators or agonists.

PPARα is another subtype of the PPAR family. PPARα is predominantly expressed in tissues catabolizing high amounts of fatty acids, such as liver, heart and brown adipose tissue. Activated PPARs form heterodimers with RXR (Retinoid X Receptor) and the heterodimer binds to a specific response element, termed PPRE (PPAR Response Element), in the regulatory regions of target genes and subsequently alters their transcription. The majority of the genes whose expression is under control of PPARα code for proteins involved in intra- and extracellular lipid metabolism, such as acyl coA oxidase, acyl-coA synthetase and apolipoproteins A-I, A-II and C-III.

Fibrates have been documented to lower plasma triglycerides and cholesterol levels and to be beneficial in the prevention of ischemic heart disease in individuals with dyslipidemia. They can also modestly decrease elevated fibrinogen and PAI-1 levels. Fibrate compounds, e.g., gemfibrozil, fenofibrate, bezafibrate, and ciprofibrate, elevate the level of plasma HDL cholesterol.

Methods and compositions presented as useful for the management of type 2 diabetes with PPAR modulators, are disclosed in WO98/05331. This document discloses in particular a composition for treating type 2 diabetes or cardiovascular disease with diabetic or pre-diabetic conditions, comprising a PPARγ agonist and a PPARα agonist. The simultaneous administration of fenofibric acid and BRL 49653 is shown to result in more pronounced effects on plasma triglyceride profiles than the administration of either agent alone.

The pharmacological profile of a PPARα activator, fenofibrate, and a PPARγ activator, rosiglitazone, was compared (E Chaput et al, Biochem Biophys Res Commun 2000 May 10;271(2):445-50) on serum parameters, target gene expression, and body weight gain in (fa/fa) fatty Zucker rats and db/db mice as well as their association in db/db mice. Fenofibrate faithfully modified the expression of PPARα responsive genes. Rosiglitazone increased adipose tissue aP2 mRNA in both models while increasing liver acyl CoA oxidase mRNA in db/db mice but not in fatty Zucker rats. Both drugs lowered serum triglycerides yet rosiglitazone markedly increased body weight gain while fenofibrate decreased body weight gain in fatty Zucker rats. KRP 297, which has been reported to be a PPARα and y co-activator, also affected serum triglycerides and insulin in fatty Zucker rats although no change in body weight gain was noted. It was further observed that in db/db mice, rosiglitazone significantly increased body weight gain by 22% while the latter was non-significantly reduced by 10% by fenofibrate, and co-administration of fenofibrate and rosiglitazone did not reduce the weight gain induced by rosiglitazone.

It therefore is an objective of the present invention to provide a method for treating weight gain associated with a treatment by rosiglitazone.

In accomplishing this and other objectives, there has been provided, in accordance with one aspect of the present invention, a therapeutic method comprised of co-administering a pharmacologically effective dose of fenofibrate and rosiglitazone, such that the weight gain associated with the rosiglitazone treatment is decreased. In this respect, it was surprisingly found that co-administration of fenofibrate and a low dose of rosiglitazone, was at least as effective as a higher dose of rosiglitazone alone in lowering blood glucose, and furthermore with less weight gain

By "PPARα agonist" is meant a compound or composition which when combined with PPARα directly or indirectly (preferably binding directly to PPARα) stimulates or increases an *in vivo* or *in vitro* reaction typical for the receptor, e.g. transcriptional regulation activity, as measured by an assay known to one skilled in the art, including, but not limited to, the "co-transfection" or "cistrans" assays described or disclosed in U.S. Patent Nos. 4,981,784, 5,071,773, 5,298,429, 5,506,102, WO89/05355, WO91/06677 WO92/05447, WO93/1123 WO93/23431, WO94/23068, WO95/18380 CA 2,034,220, and Lehmann, et al., J. Biol. Chem. 270:12953-12956 (1995). PPARα agonists may also be identified according to an assay described in US Patent 6,008,239.

Well-known PPARα agonists are fibrates compound including, but not limited to, gemfibrozil, fenofibrate, bezafibrate, clofibrate, ciprofibrate, and analogues, derivatives and pharmaceutically acceptable salts thereof. PPARα compounds are disclosed in Tontonez et al., Cell 79:1147-1156 (1994), Lehmann et al., J. Biol. Chem. 270(22):1-4, 1995, Amri et al., J. Lipid Res. 32:14491456 (1991), Kliewer et al., Proc. Natl. Acad. Sci. USA 94:4318-4323 (1997), Amri et al., J. Lipid Res. 32:1457-1463, (1991) and Grimaldi et al., Proc. Natl. Acad. Sci. USA 89:10930-10934 (1992). PPARα agonist compounds are described in US Patent 6,008,239, WO 97/27847, WO 97/27857, WO 97/28115, WO 97/28137 and WO 97/28149. Certain fibrate compounds are described in WO92/10468 and WO01/80852.

In the present invention, the fibrate is fenofibrate and pharmaceutically acceptable salts and esters of fenofibrate. Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and an enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation.

According to the present invention, the fibrate is fenofibrate which is commercially available as TricorTM capsules. Each capsule contains 67 mg of micronized fenofibrate.

By "PPARγ" agonist is meant a compound or composition which when combined with PPARγ directly or indirectly (preferably binding directly to PPARγ) stimulates or increases an *in vivo* or *in vitro* reaction typical for the receptor, e.g., transcriptional regulation activity, as measured by an assay known to one skilled in the art, including, but not limited to, the "co-transfection" or "cis-trans" assays described or disclosed in U.S. Patent Nos. 4,981,784, 5,071,773, 5,298,429, 5,506,102, WO89/05355, WO91/06677, WO 92/05447, WO93/11235 WO93/23431, WO94/23068, WO95/18380, CA 2,034,220, and Lehmann, et al., J. Biol. Chem. 270:12953-12956 (1995).

Well-known PPARγ agonists are thiazolidinedione compounds, including rosiglitazone, pioglitazone, ciglitazone, englitazone, darglitazone and analogues, derivatives and pharmaceutically acceptable salts thereof. PPARγ compounds are disclosed in Tontonez et al., Cell 79:1147-1156 (1994), Lehmann et al., J. Biol. Chem. 270(22):1-4, 1995, Amri et al., J. Lipid Res. 32:14491456 (1991), Kliewer et al., Proc. Natl. Acad. Sci. USA 94:4318-4323 (1997), Amri et al., J. Lipid Res. 32:1457-1463, (1991) and Grimaldi et al., Proc. Natl. Acad. Sci. USA 89:10930-10934 (1992).

According to the present invention, the thiazolidinedione compound is rosiglitazone.

Fenofibrate is used, in combination with rosiglitazone, to treat the weight gain associated with a rosiglitazone treatment, optionally with other therapies, by improving lipidic control.

The invention includes a method of decreasing the body weight gain associated with rosiglitazone treatment, comprising co-administering an effective dosage of fenofibrate and rosiglitazone.

In another embodiment, the invention includes a method of decreasing the weight gain associated with a rosiglitazone treatment, comprising co-administering an effective dosage of fenofibrate and rosiglitazone, where the effective dosage of fenofibrate is in the range of about 10 to about 3000 mg per day, preferably in the range of about 50 to about 300 mg per day.

In another embodiment, the effective dosage of rosiglitazone is in the range of about 0. 1 to about 100 mg per day, preferably in the range of about 0.5 to about 50 mg per day, more preferably of about 0.5 to about 10 mg per day, even more preferably of about 0.5 to about 3 mg per day, e.g. 0.5, 1.0, 1.5, 2.0, 2.5 and 3.0 mg per day.

In another embodiment, fenofibrate and rosiglitazone are administered simultaneously, in a method of decreasing the weight gain associated with the rosiglitazone treatment, comprising co-administering an effective dosage of fenofibrate and rosiglitazone.

In another embodiment of a method of decreasing the weight gain associated with the rosiglitazone treatment, fenofibrate and rosiglitazone are administered sequentially.

In another embodiment, the invention includes the use of fenofibrate, rosiglitazone and a pharmaceutically acceptable carrier for the manufacture of a medicament for decreasing the body weight gain associated with a rosiglitazone treatment.

As demonstrated in the present specification, the use of fenofibrate and rosiglitazone, has led to favorably unexpected results. Studies were designed to evaluate the effects of fenofibrate and rosiglitazone as a combination therapy, on weight gain in diabetic rats. The data showed that the weight gain associated with the rosiglitazone monotherapy was decreased when fenofibrate was administered in conjunction therewith. The data further showed that a low dose of rosiglitazone was as effective as a high dose of this compound.

As used in this application, "co-administration" means the administration of two or more compounds to the same patient, within a time period of up to about two to about twelve hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 12 hours after administration of the first compound. As described herein, the present invention encompasses co-administration of fenofibrate and rosiglitazone to a patient.

Rosiglitazone in the present invention is defined as a compound of the class of thiazolidinediones: a class of compounds which work by enhancing insulin action and promoting glucose utilization in peripheral tissue. They apparently work by enhancing insulin action and thus promoting glucose utilization in peripheral tissues, possibly by stimulating non-oxidative glucose metabolism in muscle, and suppressing gluconeogenesis in the liver.

Rosiglitazone maleate is sold under the trademark *Avandia^{™}* and is used in the management of type 2 diabetes mellitus (also known as non-insulin-dependent diabetes mellitus (NIDDM) or adult-onset diabetes).

Chemically, rosiglitazone maleate is (±)-5-[[4-[2-(methyl-2-pyridinylamino)ethoxy]phenyl]methyl]-2,4-thiazolidinedione, (Z)-2-butenedioate (1:1). The molecular formula is C₁₈H₁₉N₃O₃S·C₄H₄O₄. The molecule has a single chiral center and is present as a racemate. Due to rapid interconversion, the enantiomers are functionally indistinguishable.

Rosiglitazone is described in US Patent 5,002,953.

According to the present invention, a preparation is defined as the formulation of the active compound with encapsulating material as a carrier providing a capsule in which the active component with or without other carriers, is surrounded by a carrier, which is thus in association with it. This includes tablets, powders, capsules, pills, cachets, and lozenges which can be used as solid dosage forms suitable for oral administration.

An effective dosage is defined in the present invention as the amount of a compound that prevents or ameliorates adverse conditions or symptoms of disease(s) or disorder(s) being treated. With respect to fenofibrate and rosiglitazone, effective dosage means a pharmacological dose in the range defined above.

The present invention relates to the unexpected discovery that co-administration of fenofibrate and rosiglitazone exerts beneficial effects on the weight gain induced by a rosiglitazone treatment.

The invention includes a pharmaceutical composition comprising fenofibrate or a pharmaceutically acceptable salt thereof, rosiglitazone and a pharmaceutically acceptable carrier, for use in decreasing body weight gain associated with a rosiglitazone treatment.

As will be shown in the example, the applicant unexpectedly found that a low dosage of rosiglitazone, which does not permit a normalization of the glycemia, when associated with fenofibrate, was at least as effective as a higher dose of rosiglitazone alone in lowering blood glucose, and that furthermore it reduces significantly the weight gain induced by rosiglitazone.

In this use, the effective dosage of both agonists is as defined above.

In the use of the invention, fenofibrate and rosiglitazone can be administered simultaneously, or sequentially. In a preferred embodiment of the invention, fenofibrate and rosiglitazone are administered simultaneously, more preferably in one formulation containing both compounds.

Pharmaceutical formulations of fenofibrate and/or rosiglitazone molecules can be prepared according to known methods. The preferred route of administering fenofibrate and rosiglitazone is mucosal administration, most preferably oral administration.

For preparing pharmaceutical compositions containing fenofibrate and/or rosiglitazone, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component. In tablets, the active component is mixed with the carrier having the necessary binding properties in suitable proportions and compacted in the shape and size desired. The powders and tablets preferably contain from five or ten to about seventy percent of the active compound. Suitable carriers are magnesium carbonate, magnesium stearate, talc, sugar, pectin, dextrin, starch, gelatin, tragacanth, methylcellulose, sodium carboxymethylcellulose, a low melting wax, cocoa butter, and the like.

Liquid form preparations include solutions, suspensions, and emulsions, for example, water or water propylene glycol solutions. For parenteral injection liquid preparations can be formulated in solution e.g. in aqueous polyethylene glycol solution.

Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

A further embodiment of the invention is related to the use of fenofibrate, or fenofibrate and rosiglitazone, and a pharmaceutically acceptable carrier for the manufacture of a medicament for decreasing the body weight gain associated with a rosiglitazone treatment. Such a use is especially valuable in the treatment of type 2 diabetes mellitus.

The medicament can be the pharmaceutical preparation as defined above.

The invention is further illustrated by the following examples.

### EXAMPLE 1: Effect of fenofibrate and rosiglitazone co-administration on body weight and on glycemia

This study was designed to evaluate the effects of using a combination of rosiglitazone and fenofibrate, for the treatment of diabetes and, in addition, if this combination therapy would prevent the body weight gain that is associated with the rosiglitazone treatment.

### METHOD

### Animals:

Male homozygous Zucker rats of 9 to 11 weeks of age and their lean controls were received from Iffa-Credo (France). They were put into individual cages in a temperature-, humidity- and light- controlled room (21-23°C, 12-12h light-dark cycle). They were fed with a standard laboratory diet and had free access to water. After acclimatization, they were randomized into groups of 10, based on body weight.

The experimental groups were:
Group 1= lean rats, untreated;
Group 2 = obese rats, treated with the vehicle p.o., twice daily (at 8 a.m. and 8 p.m.);
Group 3 = obese rats, treated with Fenofibrate, 100 mg/kg, p.o., twice daily (at 8 a.m. and 8 p.m.);
Group 4 = obese rats, treated with Rosiglitazone, 0.3 mg/kg, p.o., twice daily (at 8 a.m. and 8 p.m.);
Group 5 = obese rats, treated with Rosiglitazone, 3.0 mg/kg, p.o., twice daily (at 8 a.m. and 8 p.m.);
Group 6 = obese rats, treated with Fenofibrate, 100 mg/kg and Rosiglitazone, 0.3 mg/kg, p.o., twice daily (at 8 a.m. and 8 p.m.);
Group 7 = obese rats, treated with Fenofibrate, 100 mg/kg and Rosiglitazone, 3.0 mg/kg, p.o., twice daily (at 8 a.m. and 8 p.m.).

The following parameters were monitored: glycemia (mg/dl) and body weight gain (g). The results are summarized in Tables 1 and 2.

**Table 1: results after 41 days of treatment**

| **Group** | **Glycemia** | **Body Weight** |
|---|---|---|
| **1** | 131.3 ± 4.8 | 328.4 ± 4.5 |
| **2** | 624.4 ± 32.1 | 386.2 ± 5.9 |
| **3** | 222.2 ± 53.1 | 357.0 ± 7.9 |
| **4** | 274.6 ± 56.4 | 538.5±15.8 |
| **5** | 119.6 ± 5.9 | 601.4 ± 9.3 |
| **6** | 159.1 ± 34.5 | 400.8 ± 4.3 |
| **7** | 133.3 ± 5.7 | 466.5 ± 4.6 |

**Table 2: results after 69 days of treatment**

| **Group** | **Glycemia** | **Body Weight** |
|---|---|---|
| **1** | 153.5 ± 4.6 | 371.3 ± 4.9 |
| **2** | 733.3 ± 22.8 | 397.1 ± 3.7 |
| **3** | 372.5 ± 78.5 | 380.6 ± 13.7 |
| **4** | 446.0 ± 52.4 | 615.2 ± 25.9 |
| **5** | 151.4 ± 6.5 | 721.0 ± 12.3 |
| **6** | 262.5 ± 56.9 | 435.2 ± 10.1 |
| **7** | 181.5 ± 10.4 | 548.6 ± 8.6 |

These data demonstrate the following:
- As far as body weight gain is concerned, rosiglitazone alone produced a dose-dependent increase in body weight gain. A significant reduction in body weight gain was seen upon co-administration of fenofibrate with rosiglitazone, body weight gain control being better at the low rosiglitazone dose of 0.3 mg/kg.
- As far as glycemia is concerned, (1) euglycemia was attained with 3.0 mg/kg rosiglitazone alone while the glycemia was not controlled with the 0.3 mg/kg dose between day 41 and day 69, and (2) euglycemia was perfectly maintained at day 69 with the co-administration of fenofibrate and rosiglitazone (3.0 mg/kg), and still achieved in 8 rats out of 10 when 0.3 mg/kg rosiglitazone was co-administered with fenofibrate (mean values of these 8 rats : 170.7 ± 6.3 mg/dl).

### EXEMPLE 2: Effect of a fenofibrate and rosiglitazone co-administration on glucose tolerance and insulin response

### METHOD

Obese male ZDF rats and their lean controls (GMI, Indianapolis) were fed *ad libitum* with Purina 5008. From 6.5 weeks of age, they were treated with fenofibrate (100 mg/kg, p.o., b.i.d.), rosiglitazone (0.3 mg/kg, p.o, b.i.d), the combination of both, or vehicle during 13 weeks.

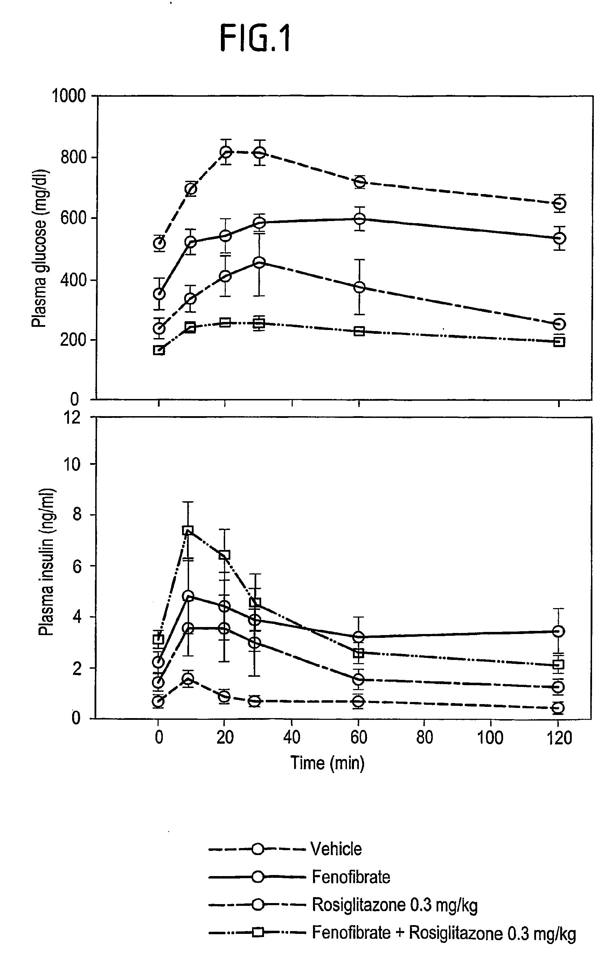

On day 98, fasted rats were submitted to an OGT test (glucose 1 g/kg as a 40% solution). Glycemia was measured by standard glucose oxidase technique and plasma insulin was determined by RIA (Linco Research) (**figure 1**).

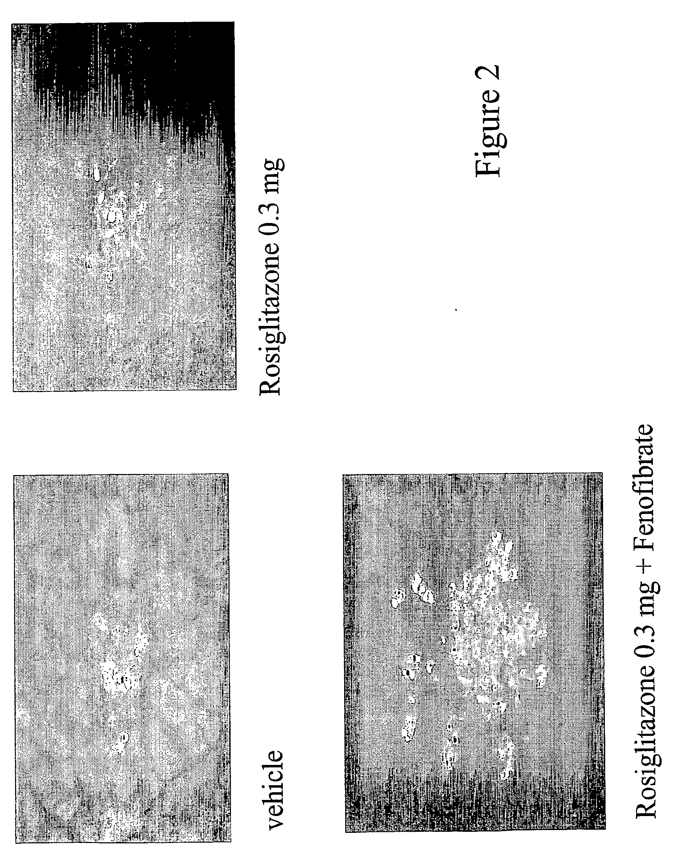

After 13 weeks of treatment, rats were sacrificed and pancreas dissected, fixed and processed for immunohistochemistry (**figure 2**). The anti-insulin serum was used and sections were photographed after indirect immunofluorescence staining.

The results observed demonstrate that the combination of a low dose of rosiglitazone with fenofibrate improved glucose tolerance and provided a perfect glycemic control with a low glycemic excursion and a swift insulin response.

Therefore, the co-administration of fenofibrate and rosiglitazone makes it possible not only to control glycemia but also to reduce the body weight gain associated with the rosiglitazone treatment. A low effective dosage of rosiglitazone is especially suitable to achieve both objectives.

## Claims

1. Use of fenofibrate and a pharmaceutically acceptable carrier in the manufacture of a medicament for decreasing body weight gain associated with a rosiglitazone treatment.

2. Use of fenofibrate, rosiglitazone and a pharmaceutically acceptable carrier in the manufacture of a medicament for decreasing body weight gain associated with a rosiglitazone treatment.

3. The use according to claim 1 or 2, wherein fenofibrate and rosiglitazone are to be administered simultaneously or sequentially.

4. The use according to one of claims 1 to 3, wherein the effective dosage of fenofibrate is in the range of 50 to 300 mg per day, and the effective dosage of rosiglitazone is in the range of about 0.5 to about 10 mg per day.

5. A pharmaceutical composition comprising fenofibrate, rosiglitazone and a pharmaceutically acceptable carrier, for use in decreasing body weight gain associated with a rosiglitazone treatment.

6. The composition according to claim 5, wherein the effective dosage of fenofibrate is in the range of 50 to 300 mg per day, and the effective dosage of rosiglitazone is in the range of about 0.5 to about 10 mg per day.

7. A combination of fenofibrate and rosiglitazone, for use in decreasing rosiglitazone-induced body weight gain.

8. The combination according to claim 7, wherein the fenofibrate and rosiglitazone are to be administered simultaneously or sequentially.

9. The combination according to claim 7 or 8, wherein the effective dosage of fenofibrate is in the range of 50 to 300 mg per day, and the effective dosage of rosiglitazone is in the range of about 0.5 to about 10 mg per day.

## Patentansprüche

1. Verwendung von Fenofibrat und einem pharmazeutisch verträglichen Carrier bei der Herstellung eines Medikaments zur Verminderung der Körpergewichtszunahme, die mit einer Rosiglitazonbehandlung verbunden ist.

2. Verwendung von Fenofibrat, Rosiglitazon und einem pharmazeutisch verträglichen Carrier bei der Herstellung eines Medikaments zur Verminderung der Körpergewichtszunahme, die mit einer Rosiglitazonbehandlung verbunden ist.

3. Verwendung nach Anspruch 1 oder 2, wobei Fenofibrat und Rosiglitazon gleichzeitig oder aufeinanderfolgend zu verabreichen sind.

4. Verwendung nach einem der Anspruche 1 bis 3, wobei die effektive Dosis von Fenofibrat im Bereich von 50 bis 300 mg pro Tag liegt und die effektive Dosis von Rosiglitazon im Bereich von 0,5 und 10 mg pro Tag liegt.

5. Pharmazeutische Zusammensetzung, umfassend Fenofibrat, Rosiglitazon und einen pharmazeutisch verträglichen Carrier, zur Verwendung bei der Verminderung der Körpergewichtszunahme, die mit einer Rosiglitazonbehandlung verbunden ist.

6. Zusammensetzung nach Anspruch 5, wobei die effektive Dosis von Fenofibrat im Bereich von 50 bis 300 mg pro Tag und die effektive Dosis von Rosiglitazon im Bereich von 0,5 bis 10 mg pro Tag liegt.

7. Kombination von Fenofibrat und Rosiglitazon zur Verwendung bei der Verminderung von Rosiglitazon-induzierter Körpergewichtszunahme.

8. Kombination nach Anspruch 7, wobei das Fenofibrat und Rosiglitazon gleichzeitig oder aufeinanderfolgend zu verabreichen sind.

9. Kombination nach Anspruch 7 oder 8, wobei die effektive Dosis von Fenofibrat im Bereich von 50 bis 300 mg pro Tag und die effektive Dosis von Rosiglitazon im Bereich von 0,5 bis 10 mg pro Tag liegt.

## Revendications

1. Utilisation du fénofibrate et d'un support pharmaceutiquement acceptable dans la fabrication d'un médicament pour réduire le gain de poids corporel associé avec un traitement à la rosiglitazone.

2. Utilisation du fénofibrate, de la rosiglitazone et d'un support pharmaceutiquement acceptable dans la fabrication d'un médicament pour réduire le gain de poids corporel associé avec un traitement à la rosiglitazone.

3. Utilisation selon la revendication 1 ou 2, où le fénofibrate et la rosiglitazone sont destinés à être administrés simultanément ou successivement.

4. Utilisation selon l'une des revendications 1 à 3, où la posologie efficace de fénofibrate est dans la plage de 50 à 300 mg par jour et la posologie efficace de rosiglitazone est dans la plage d'environ 0,5 à environ 10 mg par jour.

5. Composition pharmaceutique comprenant du fénofibrate, de la rosiglitazone et un support pharmaceutiquement acceptable pour utilisation dans la réduction du gain de poids corporel associé avec un traitement à la rosiglitazone.

6. Composition selon la revendication 5, où la posologie efficace de fénofibrate est dans la plage de 50 à 300 mg par jour et la posologie efficace de rosiglitazone est dans la plage d'environ 0,5 à environ 10 mg par jour.

7. Combinaison de fénofibrate et de rosiglitazone pour utilisation dans la réduction du gain de poids corporel induit par la rosiglitazone.

8. Combinaison selon la revendication 7, où le fénofibrate et la rosiglitazone sont destinés à être administrés simultanément ou successivement.

9. Combinaison selon la revendication 7 ou 8, où la posologie efficace de fénofibrate est dans la plage de 50 à 300 mg par jour et la posologie efficace de rosiglitazone est dans la plage d'environ 0,5 à environ 10 mg par jour.
